# FASCICULE DE BREVET EUROPEEN

(11) **EP 3 071 120 B1**
(45) Date de publication et mention de la délivrance du brevet: **20.03.2019**
(21) Numéro de dépôt: 14827464.0
(22) Date de dépôt: 17.11.2014
(51) Int. Cl.: A61B 17/068, A61B 17/128, A61B 17/28, A61B 17/00, A61B 17/29

(54) **DISPOSITIF CHIRURGICAL MULTIFONCTIONNEL RECHARGEABLE ET JETABLE**
NACHLADBARE UND MULTIFUNKTIONELLE CHIRURGISCHE EINWEGVORRICHTUNG
RELOADABLE AND DISPOSABLE MULTIFUNCTIONAL SURGICAL DEVICE

(30) Priorité: 18.11.2013 FR 1361249
(43) Date de publication de la demande: 28.09.2016
(73) Titulaire: Galiana, Jean-Marc, 69660 Collonges au Mont d'Or (FR); Ismail, Youssef, 69300 Caluire et Cuire (FR); Campbell, Phillip, Shenzen, Guangdong (CN)
(72) Inventeur: CAMPBELL, Phillip, Shenzen Guangdong (CN)
(74) Mandataire: Schmidt, Martin Peter
(86) Numéro de dépôt international: PCT/FR2014/052926
(87) Numéro de publication internationale: WO 2015/071614

(56) Documents cités:
- EP-A1- 2 537 471
- US-A1- 2002 198 537
- US-A1- 2003 040 759
- US-A1- 2006 079 913
- US-A1- 2006 097 026
- US-A1- 2008 210 738
- US-A1- 2010 292 712

## Description

### Domaine technique de l'invention

L'invention concerne un dispositif chirurgical, et plus particulièrement un dispositif destiné pour la réalisation d'opérations chirurgicales ouvertes, endoscopiques et/ou laparoscopique.

### Etat de la technique

De nombreux dispositifs chirurgicaux sont connus de l'état de la technique aussi bien en chirurgie ouverte, qu'en chirurgie endoscopique et laparoscopique. Typiquement, dans une intervention chirurgicale, chaque étape opératoire effectuée par le chirurgien nécessite l'utilisation d'un outil chirurgical différent. En effet, au cours d'une intervention, le chirurgien peut être amené à utiliser plusieurs outils différents, par exemple pour poser des clips ou agrafes de tailles et formes différentes, et très régulièrement en nombre important. Chacun de ces instruments ou applicateurs doit être stérilisé. De telles pratiques font augmenter le temps d'intervention, la complexité et le coût d'ensemble associés à ces procédures.

Plusieurs documents de l'état de la technique ont proposé de fournir un dispositif chirurgical pouvant recevoir des recharges d'un type d'outil chirurgical, notamment des applicateurs rechargeables de clips chirurgicaux ou des agrafeuses rechargeables, afin de réduire au moins en partie le coût global de ces interventions.

Le document EP 0 760 230 A1 décrit une agrafeuse chirurgicale comprenant un ensemble de poignée d'actionnement et une unité de chargement jetable contenant une pluralité d'agrafes, et une enclume installée pour être adjacente à la cartouche d'agrafes et déplaçable entre une position ouverte et une position fermée.

Le document US 6,869,435 B2 décrit un instrument chirurgical permettant d'appliquer des attaches chirurgicales lors d'une intervention chirurgicale. L'instrument comprend un manche fonctionnel à composants fonctionnels, et une cartouche d'attaches amovible dotée d'un mécanisme d'application d'attaches

Bien que comprenant des unités de chargement jetables permettant une réutilisation du manche ou de la poignée pendant une opération chirurgicale, les dispositifs de l'état de la technique montrent très souvent des dysfonctionnements et donc invalidant leur caractère réutilisable. En effet, pour de tels dispositifs rechargeables, la complémentarité des mécanismes entre celui compris dans la poignée (ou manche) et celui compris dans l'outil venant en prise dans la poignée doit être de telle sorte qu'une fois l'outil engagée dans la poignée, le dispositif doit être opérationnel immédiatement et en toute sécurité.

Un premier but selon l'invention est de proposer un dispositif chirurgical ergonomique, simple de fabrication et de fonctionnement, facile d'utilisation, et permettant de réduire le temps d'une intervention chirurgicale.

Un autre but selon l'invention est de proposer un dispositif chirurgical permettant d'accomplir plusieurs tâches différentes, et notamment pouvant agrafer, appliquer des clips chirurgicaux, disséquer, pincer ou inciser.

Un autre but selon l'invention est de proposer un dispositif chirurgical permettant de réduire le cout d'ensemble d'une procédure chirurgicale.

Un dernier but selon l'invention est de proposer un dispositif chirurgical répondant aux inconvénients des dispositifs de l'état de la technique.

### Objets de l'invention

Un premier objet de l'invention concerne un dispositif chirurgical comprenant :
- un ensemble de poignées comprenant un boîtier formant logement, un élément de poignée stationnaire, et un élément de poignée mobile pouvant être déplacé au moyen d'un mouvement d'actionnement ;
- au moins une unité de chargement jetable apte à être montée au moins partiellement de manière réversible à l'intérieur dudit boîtier formant logement, ladite unité de chargement jetable comprenant un mécanisme d'actionnement ; ledit boitier formant logement supporte :
- un premier arbre d'actionnement monté pour un mouvement longitudinal à l'intérieur du logement en réaction à un mouvement de l'élément de poignée mobile ;
- un élément de transmission monté pour actionner un mouvement longitudinal d'un second arbre d'actionnement monté à l'intérieur dudit boîtier formant logement en réaction au mouvement longitudinal du premier arbre d'actionnement ; ledit dispositif étant caractérisé en ce que ledit boîtier (10) formant logement supporte un traîneau d'actionnement principal et un traîneau d'actionnement secondaire montés pour recevoir au moins partiellement ladite au moins une unité de chargement jetable, lesdits traîneaux d'actionnement étant fixés respectivement aux arbres d'actionnement pour activer le mécanisme d'actionnement de l'unité de chargement jetable en réaction à leurs mouvements longitudinaux.

Les arbres d'entraînement comprennent une crémaillère dentée, l'élément de transmission venant en prise avec chacune desdites crémaillères.

Avantageusement, le traîneau d'actionnement secondaire est contenu dans le traîneau d'actionnement principal et est apte à se déplacer longitudinalement au moyen d'un élément de guidage.

De préférence, le logement supporte en outre un élément de rétractation relié à son extrémité proximale au premier arbre d'actionnement et à son extrémité distale à un élément de support fixe.

Avantageusement, les arbres d'entraînements et les traîneaux d'actionnement forment chacun respectivement une seule pièce préformée.

L'unité de chargement jetable comprend préférentiellement un élément de fixation venant en prise au moins partiellement avec la partie proximale du corps de l'unité de chargement jetable.

Le boitier formant logement comprend avantageusement un élément de déverrouillage, ledit élément de déverrouillage comprenant une portion en forme de saillie s'étendant au moins partiellement dans le boitier afin de coopérer par encliquetage avec le rebord d'un élément de blocage.

Un autre objet concerne un kit comprenant un dispositif selon chirurgical selon l'invention, et une pluralité d'unités de chargement jetables sous la forme d'applicateurs de clips chirurgicaux comprenant chacun au moins une rangée linéaire de clips chirurgicaux.

Un autre objet concerne un kit comprenant un dispositif selon l'invention et une pluralité d'unités de chargements jetables de préférence sélectionné dans le groupe formé par :
- les applicateurs de clips chirurgicaux ;
- les agrafeuses ;
- les outils incisifs, notamment les lames incisives.

### Description des figures

La figure 1 est une représentation en perspective d'un mode de réalisation du dispositif chirurgical selon l'invention dans lequel une unité de chargement jetable **40** est fonctionnellement engagée dans la partie distale du boitier **10** formant logement. La flèche « C » illustre la course d'actionnement de la poignée mobile **13.**
La figure 2a est une vue en perspective de la section transversale de l'ensemble de poignées **30** du dispositif chirurgical.
La figure 2b est une vue latérale en section transversale de l'ensemble de poignées **30** du dispositif chirurgical selon l'invention.
La figure 3a est une représentation en perspective du mécanisme d'actionnement supporté dans le logement **10** de l'ensemble de poignées **30.**
La figure 3b est une représentation en vue éclatée du mécanisme d'actionnement de la figure 3a.
La figure 4a est une vue en perspective d'une unité de chargement jetable **40** apte à être fonctionnellement engagée avec l'ensemble de poignées **30** du dispositif chirurgical selon l'invention.
La figure 4b est une représentation en vue éclatée de l'unité de chargement jetable **40** de

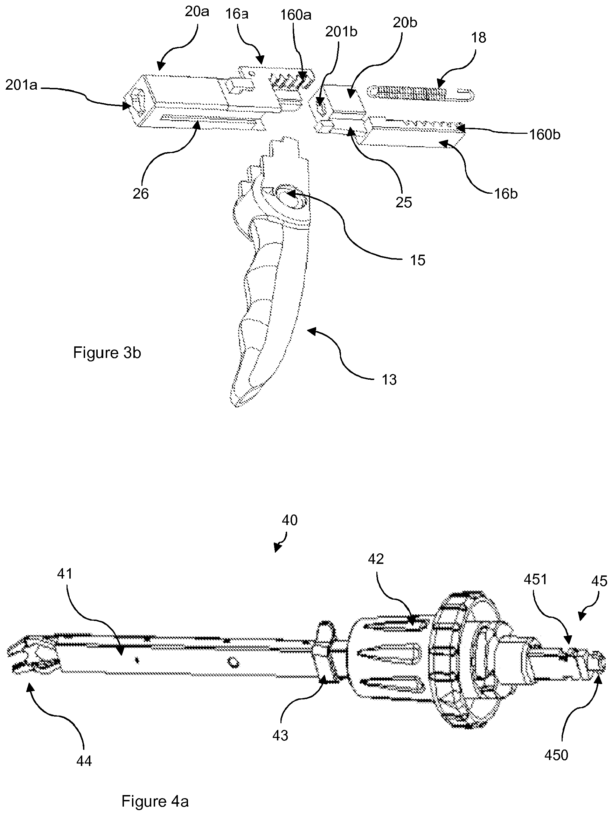

la figure 4a.

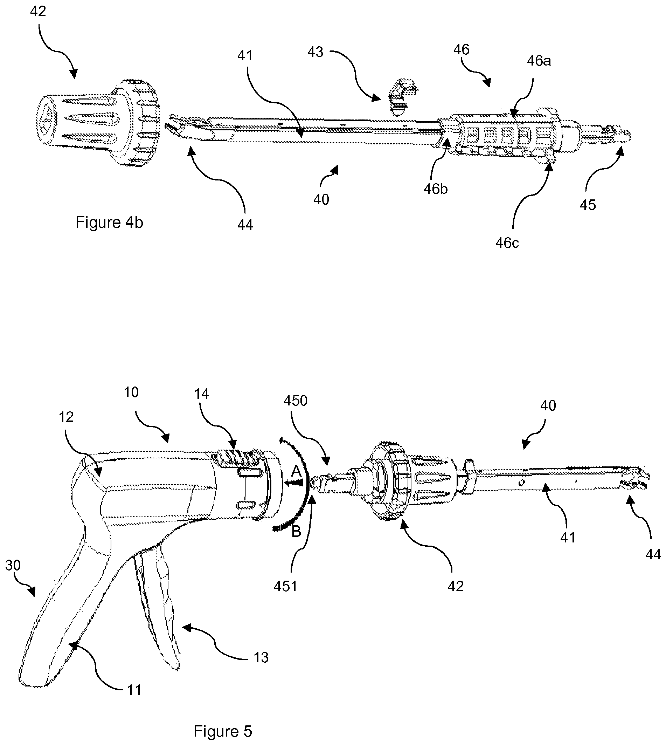

La figure 5 est une représentation en perspective du mécanisme de mis en engagement d'une unité de chargement jetable **40** sur l'ensemble de poignées **30** du dispositif chirurgical selon l'invention. La flèche « A » indique un mouvement de translation longitudinal dans le sens proximal de l'unité de chargement jetable **40** ; la flèche « B » indique un mouvement de rotation de l'unité de chargement jetable **40.**

**Liste des repères :**

| | |
|---|---|
| 1 | Dispositif |
| 10 | Boitier |
| 11 | Elément de poignée stationnaire |
| 12 | Portion de fût |
| 13 | Elément de poignée mobile |
| 14 | Elément de déverrouillage |
| 15 | Pivot |
| 16a | Premier arbre d'actionnement |
| 16b | Second arbre d'actionnement |
| 17 | Elément de transmissions |
| 18 | Elément de traction |
| 19 | Elément de support |
| 20a | Traîneau d'actionnement principal |
| 20b | Traîneau d'actionnement secondaire |
| 25 | Rail de guidage |
| 26 | Pièce de guidage |
| 30 | Ensemble de poignées |
| 31 | Ouverture |
| 40 | Unité de chargement jetable |
| 41 | Corps |
| 42 | Elément de fixation |
| 43 | Elément de maintien |
| 44 | Ensemble d'outils |
| 45 | Portion proximale |
| 46 (46a, 46b) | Elément de blocage |
| 46c | Rebord périphérique de l'élément de blocage |
| 160a,160b | Crémaillères dentées |
| 201a,201b | Ouvertures distales |
| 450 | Canal dentaire inférieur |
| 451 | Cloison horizontale |

### Description détaillée de l'invention

### 1. Définitions

Le terme « proximal » se réfère à l'extrémité du dispositif (ou appareil) qui est la plus proche de l'opérateur ; le terme « distal » se réfère à l'extrémité du dispositif (ou appareil) qui est la plus éloignée de l'opérateur.

### 2. Description

Des modes de réalisation préférés du dispositif chirurgical multifonctionnel seront maintenant décrits en détail en se reportant aux figures. On comprendra que plusieurs modifications peuvent être apportées aux modes de réalisation divulgués ci-après. La description ne doit pas être comprise comme une limitation de l'invention, mais uniquement comme des exemplifications de modes de réalisation préférés. Bien entendu, l'homme du métier peut envisager d'autres modifications.

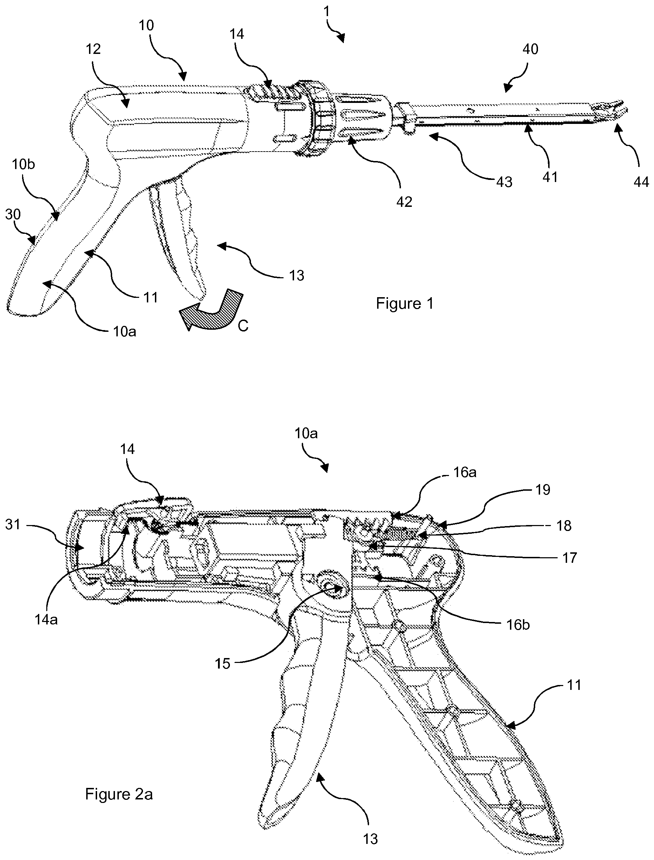

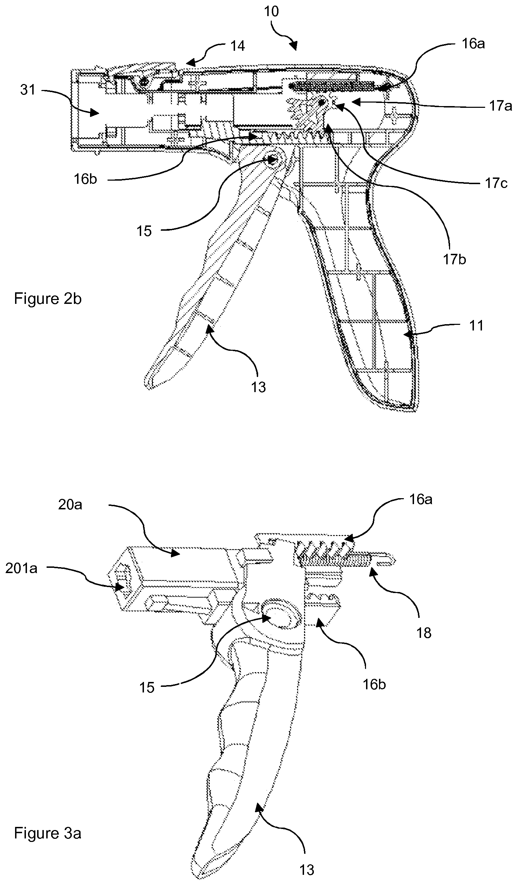

En se rapportant aux figures 1, 2a et 2b, l'ensemble de poignées **30** comprend un élément de poignée stationnaire **11** et un élément de poignée mobile **13,** et comprend également un boîtier **10** formant logement qui est constitué de préférence de demi-sections de boîtier moulées **10a** et **10b,** qui forment l'élément de poignée stationnaire **11** et une portion de fût **12** de l'ensemble de poignées **30.** L'ensemble formant poignées **30** comprend une ouverture **31** apte à accueillir une portion d'une unité de chargement jetable **40,** comme il sera décrit plus en détail ci-après. L'élément de poignée mobile **13** est supporté d'une manière pivotante entre les demi-sections de boîtier **10a** et **10b** autour du pivot **15** (figure 2a). Un élément de sollicitation (non représenté sur les figures), qui est de préférence un ressort de torsion, sollicite la poignée mobile **13** au loin de la poignée stationnaire **11.** Dans la portion de fût **12** sont supportés deux arbres d'entraînement **16a,16b** comprenant chacun une crémaillère dentée **160a,160b.** Un élément de transmission **17** (figure 2b) vient en prise avec chacune des deux crémaillères **160a,160b** des arbres d'entraînement **16a,16b** et est monté d'une manière pivotante autour d'un pivot **17c.** Plus particulièrement, l'élément de transmission **17** est constitué d'une portion de roue écrantée **17a** apte à coopérer avec la crémaillère **160a** du premier arbre d'entraînement, et une seconde portion de roue écrantée **17b** apte à coopérer avec la crémaillère **160b** du second arbre d'entraînement **16b.** L'axe de rotation des deux portions de roues écrantées **17a,17b** est situé au niveau du pivot **17c.**

La poignée mobile **13** peut pivoter pour faire avancer le premier arbre d'actionnement **16a** linéairement dans la direction distale et entraîner l'élément de transmission **17** en contact avec la crémaillère dentée **160a** dudit premier arbre d'actionnement **16a.** La rotation de l'élément de transmission **17** permet le déplacement du second arbre d'actionnement **16b** linéairement dans la direction proximale.

Le mécanisme de rétractation qui comprend un élément de rétractation **18** peut être avantageusement relié à l'extrémité proximale du premier arbre d'actionnement **16a** par un ressort de traction **18.** Aussi, le ressort de traction **18** est relié à un élément de support fixe **19,** de tel sorte que ledit ressort **18** est configuré pour effectuer un mouvement de translation longitudinale dans la direction distale ou proximale.

En se rapportant aux figures 3a et 3b, le premier arbre d'entraînement **16a** est relié à un traîneau d'actionnement principal **20a** comprenant à l'extrémité distale une ouverture **201a** coopérant avec la partie proximale de l'unité de chargement jetable **40** comme il sera décrit en détail plus loin dans la description. Le second arbre d'entraînement **16b** est quant à lui relié au traîneau d'actionnement secondaire **20b** au moyen d'un élément de guidage **25.** Le traîneau d'actionnement **20b** comprend à son extrémité distale une ouverture **201b** coopérant avec la partie proximale de l'unité de chargement jetable **40.** Dans un mode de réalisation préféré, l'ouverture **201b** du traîneau d'actionnement secondaire **20b** est de diamètre inférieur à celle du traîneau d'actionnement principal **20a.**

Tel que mieux illustré en figure 3b, le traîneau d'actionnement secondaire **20b** est contenu dans le traîneau d'actionnement **20a,** et est apte à se déplacer longitudinalement au moyen d'un rail de guidage **25** permettant de déplacer d'une manière coulissante la pièce de guidage **26** sur le rail de guidage **25.**

Dans un mode de réalisation préféré, les arbres d'entrainement **16a,16b** et les traîneaux d'actionnement **20b,20a** forment chacun respectivement une seule pièce préformée par moulage (figure 3b).

En se reportant aux modes de réalisation des figures 3a et 3b, un mécanisme de fixation de l'unité de chargement jetable **40** s'étend dans le boîtier **10** de l'ensemble de poignées **30.** Le mécanisme de fixation comprend un premier traîneau d'actionnement **20a** de section rectangulaire qui est supporté d'une manière coulissante avec le premier arbre d'entraînement **16a,** et un traîneau d'actionnement secondaire **20b** de section rectangulaire supporté d'une manière coulissante au second arbre d'entraînement **16b.** Les deux ouvertures distales **201a,201b** des traîneaux d'actionnement **20a,20b** sont de forme sensiblement circulaire, et comprennent en outre deux évidements supplémentaires situés de part et d'autre de l'ouverture permettant le passage de l'extrémité proximale de l'unité de chargement jetable **40** au niveau de sa partie proximale **45.**

En se reportant aux modes de réalisation des figures 1, 2 et 3, le boitier **10** renferme entre autres le premier arbre d'entraînement **16a,** le premier traîneau d'actionnement **20a,** le second arbre d'entraînement **16b** et le traîneau d'actionnement secondaire **20b,** ainsi que l'élément de transmission **17** et l'élément de traction **18** ; cela représente un mode de réalisation avantageux.

En se reportant à la figure 4, l'unité de chargement jetable **40** comprend une portion proximale **45** apte à venir relâchablement prise avec l'extrémité distale de l'ensemble de poignées **30** (figure 1). Un mécanisme d'actionnement (non représenté dans les figures) compris dans le corps **41** de l'unité de chargement jetable **40** est configuré pour actionner l'ensemble d'outils **44.** La portion proximale **45** de l'unité de chargement comporte une première paire de crochets **450** et une deuxième paire de crochets **451** permettant la fixation de l'unité de chargement jetable **40** sur l'ensemble formant poignées **30,** à l'extrémité distale de la portion de fût **12** du boitier **10,** comme il sera décrit plus en détail ci-après. L'unité de chargement jetable **40** comprend en outre un élément de blocage **46** constitué de préférence de demi-sections moulées **46a** et **46b** permettant le blocage et l'alignement de l'ensemble de montage compris dans le corps de l'unité de chargement. L'extrémité proximale de l'élément de blocage **46** comprend un rebord **46c** apte à coopérer avec l'élément de déverrouillage **14** par encliquetage. Plus particulièrement, l'élément de déverrouillage comprend une portion en forme de saillie **14a** située dans le logement **10** du dispositif coopérant avec le rebord **46c** de l'élément de blocage.

Dans un mode de réalisation avantageux, l'unité de chargement jetable **40** comprend un élément de fixation **42** venant en prise au moins partiellement avec la partie proximale du corps **41** de l'unité de chargement jetable, et « entourant » au moins partiellement l'élément de blocage **46.** L'élément de fixation permet de conserver l'alignement de l'ensemble des pièces constitutives du corps **41,** et plus particulièrement l'ensemble du mécanisme d'actionnement compris dans ledit corps **41.** En effet, pour fonctionner correctement, l'ensemble des pièces constitutives de l'unité de chargement **40** doivent être parfaitement alignées les unes par rapports aux autres, avec des tolérances de seulement quelques dixièmes de millimètres, pour actionner l'ensemble du dispositif.

En se reportant aux figures 4 et 5, pour utiliser un mode de réalisation du dispositif chirurgical selon l'invention, une unité de chargement jetable **40** est d'abord fixée à l'extrémité distale de l'ensemble formant poignées **30.** Pour fixer l'unité de chargement jetable **40** sur l'ensemble formant poignée **30,** la portion proximale **45** de l'unité de chargement est insérée dans le l'ouverture **30a** de l'ensemble formant poignée **30,** la première paire de crochets **450** est amenée à coulisser longitudinalement dans l'extrémité distale du boitier **10** dans la direction indiquée par la flèche « A » sur la figure 5, de telle sorte que la première paire de crochets **450** s'engage dans un premier temps à travers le premier traîneau d'actionnement **20a** via l'ouverture distale **201a,** puis dans un second temps à travers le second traîneau d'actionnement **20b** via l'ouverture distale **201b.** Lorsque la première paire de crochets **450** vient en engagement dans le traîneau d'actionnement **20b,** la seconde paire de crochets **451** s'engage à travers le traîneau d'actionnement **20a** via l'ouverture distale **201a.** Simultanément, l'extrémité proximale de l'élément de blocage **46** comprend coopère un avec l'élément de déverrouillage **14** par encliquetage.

Une fois les deux paires de crochets **450,451** engagées, l'unité de chargement jetable **40** est amenée à tourner dans la direction indiquée par la flèche « B » sur la figure 5 pour amener les paires de crochets **450,451** en prise avec les épaulements internes des extrémités distales des traîneaux d'actionnement **20a,20b.** Il faut noter que lorsque l'unité de chargement jetable est amenée à tourner, un son de click audible est produit indiquant que l'unité de chargement jetable **40** est fixée correctement au logement **10.**

Lorsque l'unité de chargement **40** jetable est fixée au boitier **10,** l'outil **45** peut être utilisé et positionné pour délivrer une pluralité de clips. Pour ce faire, la poignée mobile **13** est déplacée dans la direction indiquée par la flèche « C » sur la figure 1 contre la sollicitation du ressort de torsion pour amener à faire avancer le premier arbre d'actionnement **16a** linéairement dans la direction distale et pour faire avancer le second arbre d'actionnement **16b** linéairement dans la direction proximale au moyen de l'élément de transmission **17.**

Le déplacement des arbres d'actionnement **16a,16b** entraînent le déplacement de leurs traîneaux d'actionnement respectif **20a,20b.** L'unité de chargement jetable **40** étant fixé sur la poignée **30,** le déplacement du traîneau d'actionnement **20b** entraîne avec lui le déplacement dans la direction proximale de la première paire de crochets **450** qui est solidaire d'une première plaque (non représentée sur les figures). La première plaque se déplace ainsi longitudinalement dans la direction proximale jusqu'à venir en prise avec un clip « n-1 » stocké dans le corps **43** de l'unité de chargement mobile **40.** Simultanément, le déplacement du traîneau d'actionnement **20a** entraîne avec lui le déplacement dans la direction distale de la seconde paire de crochets **451** qui est-elle solidaire d'une deuxième plaque (non représentée sur les figures) permettant de déplacer un clip « n » dans la direction distale, i.e. en direction de l'ensemble d'outil **44.** De plus, associé au déplacement de la paire de crochets **451,** une troisième plaque (non représentée sur les figures) est entraînée dans la direction distale vers l'ensemble d'outil **44** permettant l'actionnement de ce dernier, notamment permettant dans le cas d'un applicateur de clip de refermer les mâchoires de l'ensemble d'outil autour du clip « n ».

De manière avantageuse, l'unité de chargement jetable **40** comprend en outre un élément de maintien **43** situé au moins partiellement sur le corps **41** de l'unité de chargement pour conserver l'alignement des clips compris dans le corps **41** avant l'utilisation du dispositif, notamment lorsque le dispositif est transporté ou stocké. Lors de l'utilisation du dispositif **1,** l'élément de maintien **43** doit être retiré pour activer les différents mécanismes de délivrance des clips.

En se rapportant à nouveau à la figure 1, l'unité de chargement jetable **40** invalidée ou verrouillée peut être retirée du boîtier **10** en exerçant une pression sur l'élément de déverrouillage **14,** puis en faisant tourner l'unité de chargement jetable **40** dans la direction opposée à la direction indiquée par la flèche « B » sur la figure 5 pour désengager les paires de crochets **450,451** sur les épaulement internes des traîneaux d'actionnement **20a,20b.** Après la rotation, l'unité de chargement jetable **40** peut être amenée à coulisser dans la direction opposée à celle indiquée par la flèche « A » pour détacher le boitier **10** de l'unité de chargement jetable **40.** Par la suite, des unités de chargement jetables additionnelles peuvent être fixées à l'extrémité distale du boitier **10,** comme il a été décrit ci-dessus, pour exécuter la même opération, i.e. la délivrance de clips, ou pour exécuter d'autres tâches, par exemple l'agrafage de tissus, une incision, une opération de section.

Le dispositif selon l'invention comprend donc un mécanisme simplifié réduisant considérablement les besoins d'outillage et d'assemblage, tout en offrant une très bonne fiabilité opérationnelle, et à moindre coût. Pour fonctionner correctement, le dispositif selon l'invention nécessite des tolérances générales faibles. A titre d'illustration, les tolérances générales concernant le dimensionnement des clips sont les suivantes : ± 0,30 mm sur toutes les faces planes du clip, ± 0,4 mm pour la hauteur, ± 0,40mm largeur du clip entre les mâchoires d'un ensemble d'outils.

L'unité de chargement jetable **40** peut comprendre au moins une rangée linéaire d'au plus trente clips, de préférence d'au plus vingt clips. Ces clips sont utilisés pour ligaturer des vaisseaux sanguins, notamment des artères, ou avant de les sectionner ou d'assembler des tissus organiques ou synthétiques. Les clips peuvent être résorbables ou non résorbables, dans ce dernier cas les clips sont de préférence en titane et de forme sensiblement en U. De plus, la longueur de la rangée linéaire de clips chirurgicaux ou d'agrafes peut être modifiée pour répondre aux besoins d'une intervention chirurgicale particulière.

Bien évidemment, plusieurs modifications peuvent être apportées aux modes de réalisation décrits ci-avant. Par exemple, le dispositif selon l'invention n'applique pas nécessairement que des clips chirurgicaux (ou attaches chirurgicales) mais peut appliquer également des agrafes, par exemple pour l'ablation de tout ou partie d'un organe et/ou pour permettre la réalisation d'anastomose. Ainsi le dispositif peut être utilisé avec des unités de chargement jetables conçues pour appliquer des rangées linéaires d'agrafes peut être utilisé pour activer des unités jetables de chargement contenant des agrafes individuelles.

Le dispositif selon l'invention peut également supporter en son logement une unité de chargement jetable dont la partie d'extrémité distale supporte une lame configurée pour réaliser des incisions, par exemple une incision dans un tissu corporel agrafé.

## Revendications

1. Dispositif chirurgical (1) comprenant :
- un ensemble de poignées (30) comprenant un boîtier (10) formant logement, un élément de poignée stationnaire (11), et un élément de poignée mobile (13) pouvant être déplacé au moyen d'un mouvement d'actionnement ;
- au moins une unité de chargement jetable (40) apte à être montée au moins partiellement de manière réversible à l'intérieur dudit boîtier (10) formant logement, ladite unité de chargement jetable comprenant un mécanisme d'actionnement; ledit boitier (10) formant logement supporte :
- un premier arbre d'actionnement (16a) monté pour un mouvement longitudinal à l'intérieur du logement en réaction à un mouvement de l'élément de poignée mobile (13) ;
- un élément de transmission (17) monté pour actionner un mouvement longitudinal d'un second arbre d'actionnement (16b) monté à l'intérieur dudit boîtier formant logement (10) en réaction au mouvement longitudinal du premier arbre d'actionnement (16a) ; ledit dispositif étant **caractérisé en ce que** ledit boîtier (10) formant logement supporte un traîneau d'actionnement principal (20a) et un traîneau d'actionnement secondaire (20b) montés pour recevoir au moins partiellement ladite au moins une unité de chargement jetable (40), lesdits traîneaux d'actionnement (20a, 20b) étant fixés respectivement aux arbres d'actionnement (16b,16a) pour activer le mécanisme d'actionnement de l'unité de chargement jetable (40) en réaction à leurs mouvements longitudinaux.

2. Dispositif selon la revendication 1, **caractérisé en ce que** les arbres d'entraînement (16a,16b) comprennent une crémaillère dentée (160a,160b), l'élément de transmission (17) venant en prise avec chacune desdites crémaillères (160a,160b).

3. Dispositif selon la revendication 1 ou 2, **caractérisé en ce que** traîneau d'actionnement secondaire (20b) est contenu dans le traîneau d'actionnement principal (20a) et est apte à se déplacer longitudinalement au moyen d'un élément de guidage (25).

4. Dispositif selon l'une quelconque des revendications 1 à 3, **caractérisé en ce que** le logement supporte en outre un élément de rétractation (18) relié à son extrémité proximale au premier arbre d'actionnement (16a) et à son extrémité distale à un élément de support fixe (19).

5. Dispositif selon l'une quelconque des revendications 1 à 4, **caractérisé en ce que** les arbres d'entraînements (16a,16b) et les traîneaux d'actionnement (20b,20a) forment chacun respectivement une seule pièce préformée.

6. Dispositif selon la revendication 5, **caractérisé en ce que** les arbres d'entraînements (16a,16b) et les traîneaux d'actionnement (20b,20a) forment chacun respectivement une seule pièce préformée par moulage.

7. Dispositif selon l'une quelconque des revendications 1 à 6, **caractérisé en ce que** ladite au moins une unité de chargement jetable (40) comprend un élément de fixation (42) venant en prise au moins partiellement avec la partie proximale du corps (41) de l'unité de chargement jetable (40).

8. Dispositif selon l'une quelconque des revendications 1 à 7, **caractérisé en ce que** le boitier (10) formant logement comprend un élément de déverrouillage (14), ledit élément de déverrouillage comprenant une portion en forme de saillie s'étendant au moins partiellement dans le boitier (10) afin de coopérer par encliquetage avec le rebord d'un élément de blocage (46).

9. Kit comprenant un dispositif selon l'une quelconque des revendications 1 à 8, et une pluralité d'unités de chargement jetables (40) sous la forme d'applicateurs de clips chirurgicaux comprenant chacun au moins une rangée linéaire de clips chirurgicaux.

10. Kit comprenant un dispositif selon l'une quelconque des revendications 1 à 8, et une pluralité d'unités de chargements jetables (40) de préférence sélectionné dans le groupe formé par :
- les applicateurs de clips chirurgicaux ;
- les agrafeuses ;
- les outils incisifs, notamment les lames incisives.

## Patentansprüche

1. Chirurgische Vorrichtung (1), die Folgendes umfasst:
- eine Einheit von Griffen (30), die ein Gehäuse (10) umfasst, das eine Aufnahme bildet, ein stationäres Griffelement (11) und ein bewegliches Griffelement (13), das mittels einer Betätigungsbewegung verlagert werden kann;
- mindestens eine Einweg-Ladeeinheit (40), die geeignet ist, um mindestens teilweise auf umkehrbare Art in dem Inneren des Gehäuses (10), das eine Aufnahme bildet, montiert zu werden, wobei die Einweg-Ladeeinheit einen Betätigungsmechanismus umfasst;
wobei das Gehäuse (10), das eine Aufnahme bildet, Folgendes trägt:
- eine erste Betätigungswelle (16a), die für eine Längsbewegung in dem Inneren der Aufnahme als Reaktion auf eine Bewegung des beweglichen Griffelements (13) montiert ist;
- ein Übertragungselement (17), das montiert ist, um eine Längsbewegung einer zweiten Betätigungswelle (16b), die in dem Inneren des Gehäuses (10), das eine Aufnahme bildet, montiert ist, als Reaktion auf die Längsbewegung der ersten Betätigungswelle (16a) zu betätigen;
wobei die Vorrichtung **dadurch gekennzeichnet ist, dass** das Gehäuse (10), das eine Aufnahme bildet, einen Hauptbetätigungswagen (20a) und einen Nebenbetätigungswagen (20b) trägt, die montiert sind, um mindestens teilweise die mindestens eine Einweg-Ladeeinheit (40) aufzunehmen, wobei die Betätigungswagen (20a, 20b) jeweils an den Betätigungswellen (16b, 16a) befestigt sind, um den Betätigungsmechanismus der Einweg-Ladeeinheit (40) als Reaktion auf ihre Längsbewegungen zu aktivieren.

2. Vorrichtung nach Anspruch 1, **dadurch gekennzeichnet, dass** die Antriebswellen (16a, 16b) eine Zahnstange (160a, 160b) umfassen, wobei das Übertragungselement (17) mit einer der Zahnstangen (160a, 160b) in Eingriff kommt.

3. Vorrichtung nach Anspruch 1 oder 2, **dadurch gekennzeichnet, dass** der Nebenbetätigungswagen (20b) in dem Hauptbetätigungswagen (20a) enthalten und geeignet ist, um sich längs mittels eines Führungselements (25) zu bewegen.

4. Vorrichtung nach einem der Ansprüche 1 bis 3, **dadurch gekennzeichnet, dass** die Aufnahme außerdem ein Rückziehelement (18) trägt, das an seinem proximalen Ende mit der ersten Betätigungswelle (16a) und an seinem distalen Ende mit einem stationären Tragelement (19) verbunden ist.

5. Vorrichtung nach einem der Ansprüche 1 bis 4, **dadurch gekennzeichnet, dass** die Antriebswellen (16a, 16b) und die Betätigungswagen (20b, 20a) jeweils ein einziges vorgeformtes Teil bilden.

6. Vorrichtung nach Anspruch 5, **dadurch gekennzeichnet, dass** die Antriebswellen (16a, 16b) und die Betätigungswagen (20b, 20a) jeweils ein einziges durch Formen vorgeformtes Teil bilden.

7. Vorrichtung nach einem der Ansprüche 1 bis 6, **dadurch gekennzeichnet, dass** die mindestens eine Einweg-Ladeeinheit (40) ein Befestigungselement (42) umfasst, das mindestens teilweise mit dem proximalen Teil des Körpers (41) der Einweg-Ladeeinheit (40) in Eingriff kommt.

8. Vorrichtung nach einem der Ansprüche 1 bis 7, **dadurch gekennzeichnet, dass** das Gehäuse (10), das eine Aufnahme bildet, ein Entriegelungselement (14) umfasst, wobei das Entriegelungselement einen Abschnitt in vorragender Form umfasst, der sich mindestens teilweise in das Gehäuse (10) erstreckt, um durch Einrasten mit der Krempe eines Blockierungselements (46) zusammenzuwirken.

9. Satz, der eine Vorrichtung nach einem der Ansprüche 1 bis 8 umfasst, sowie eine Vielzahl von Einweg-Ladeeinheiten (40) in der Form chirurgischer Clipsapplikatoren, die jeweils mindestens eine lineare Reihe chirurgischer Clips umfassen.

10. Satz, der eine Vorrichtung nach einem der Ansprüche 1 bis 8 umfasst, sowie eine Vielzahl von Einweg-Ladeeinheiten (40), die bevorzugt aus der Gruppe ausgewählt sind, die aus folgenden besteht:
- Applikatoren chirurgischer Clips;
- Heftmaschinen;
- scharfen Werkzeugen, insbesondere scharfen Klingen.

## Claims

1. Surgical device (1) comprising:
- a handle assembly (30) comprising a casing (10) forming a housing, a stationary handle element (11), and a movable handle element (13) that can be moved by means of an actuating movement;
- at least one disposable loading unit (40) suitable for being mounted at least partially in a reversible manner inside said casing (10) forming a housing, said loading unit comprising an actuation mechanism;
said casing (10) forming a housing supports:
- a first actuation shaft (16a) mounted to make a longitudinal movement inside the housing in response to the movement of the movable handle element (13);
- a transmission element (17) mounted to actuate a longitudinal movement of a second actuation shaft (16b) mounted inside said casing forming a housing (10) in response to the longitudinal movement of the first actuation shaft (16a);
said device being **characterised in that** said casing (10) forming a housing supports a main actuation sled (20a) and a secondary actuation sled (20b) mounted to at least partially receive said at least one disposable loading unit (40), said actuation sleds (20a, 20b) being secured respectively to the actuation shafts (16b, 16a) in order to activate the actuation mechanism of the disposable loading unit (40) in response to the longitudinal movements thereof.

2. Device according to claim 1, **characterised in that** the drive shafts (16a, 16b) comprise a toothed rack (160a, 160b), the transmission element (17) engaging with each of said racks (160a, 160b).

3. Device according to claim 1 or 2, **characterised in that** the secondary actuation sled (20b) is contained in the main actuation sled (20a) and is suitable for moving longitudinally by means of a guiding element (25).

4. Device according to any one of claims 1 to 3, **characterised in that** the housing further supports a retraction element (18) connected at the proximal end thereof to the first actuation shaft (16a) and at the distal end thereof to a fixed support element (19).

5. Device according to any one of claims 1 to 4, **characterised in that** the drive shafts (16a, 16b) and the actuation sleds (20b, 20a) each form a single preformed part respectively.

6. Device according to claim 5, **characterised in that** the drive shafts (16a, 16b) and the actuation sleds (20b, 20a) each respectively form a single preformed part by moulding.

7. Device according to any one of claims 1 to 6, **characterised in that** said disposable loading unit (40) comprises a fastening element (42) engaging at least partially with the proximal part of the body (41) of the disposable loading unit (40).

8. Device according to any one of claims 1 to 7, **characterised in that** the casing (10) forming a housing comprises an unlocking element (14), said unlocking element comprising a portion in the form of a projection extending at least partially into the casing (10) in order to engage by locking with the edge of a locking element (46).

9. Kit comprising a device according to any one of claims 1 to 8, and a plurality of disposable loading units (40) in the form of surgical clip applicators each comprising at least one linear row of surgical clips.

10. Kit comprising a device according to any one of claims 1 to 8, and a plurality of disposable loading units (40) preferably selected in the group formed by:
- surgical clip applicators;
- staplers;
- incision tools, notably incision blades.
